# FASCICULE DE BREVET EUROPEEN

(11) **EP 2 941 303 B1**
(45) Date de publication et mention de la délivrance du brevet: **10.05.2017**
(21) Numéro de dépôt: 13803180.2
(22) Date de dépôt: 26.11.2013
(51) Int. Cl.: A61Q 17/04

(54) **NOUVELLES ÉMULSIONS HUILE-DANS-EAU RICHES EN SELS, À VISCOSITÉ ÉLEVÉE ET STABLES AU COURS DU TEMPS**
NEUARTIGE ÖL-IN-WASSER-EMULSIONEN REICH AN SALZEN MIT HOHER VISKOSITÄT UND STABILITÄT
NOVEL OIL-IN-WATER EMULSIONS RICH IN SALTS, HAVING HIGH VISCOSITY AND STABLE OVER TIME

(30) Priorité: 04.01.2013 FR 1350065
(43) Date de publication de la demande: 11.11.2015
(73) Titulaire: Societe D Exploitation De Produits Pour Les Industries Chimiques Seppic, 75007 Paris (FR)
(72) Inventeur: MERAT, Emmanuelle, F-81440 Lautrec (FR)
(74) Mandataire: Conan, Philippe Claude
(86) Numéro de dépôt international: PCT/FR2013/052857
(87) Numéro de publication internationale: WO 2014/106698

(56) Documents cités:
- EP-A1- 1 203 789
- FR-A1- 2 910 899

## Description

L'invention a pour objet de nouvelles émulsions huile-dans-eau, ainsi que leurs utilisations en cosmétique et en pharmacie.

Les compositions cosmétiques se présentant sous la forme d'émulsions huile-dans-eau commercialisées par l'industrie cosmétique et par l'industrie pharmaceutique comprennent très régulièrement des polymères épaississants synthétiques pour augmenter la viscosité desdites émulsions huile-dans-eau qui peuvent se présenter sous la forme de crèmes, de laits, et qui sont appliquées directement sur la peau.

Ces polymères épaississants synthétiques permettent d'épaissir les phases aqueuses présentes dans lesdites émulsions huile-dans-eau, procurant ainsi soit l'obtention de la consistance désirée soit un effet de stabilisation de ladite émulsion.

Les polymères épaississants synthétiques actuellement utilisés en ces domaines, se présentent sous deux formes physiques, la forme poudre et la forme liquide pour laquelle le polymère est préparé par polymérisation radicalaire en émulsion inverse à l'aide d'agents tensioactifs, et que l'on appelle couramment latex inverse.

Parmi les polymères épaississants synthétiques se présentant sous la forme d'une poudre les plus connus, on peut citer les polymères à base d'acide acrylique ou les copolymères à base d'acide acrylique et de ses esters. On citera par exemple, les polymères commercialisés sous le nom de marque, CARBOPOL™ et PEMULEN™. Ils sont décrits notamment dans les brevets américains US 5,373,044, US 2,798,053 et dans le brevet européen EP 0 301 532.

En cosmétique, on utilise aussi et toujours sous forme de poudre, des homopolymères ou des copolymères à base d'acide 2-acrylamido-2-méthyl-propane sulfonique et/ou de ses sels. Ces polymères épaississants sont commercialisés sous le nom de marque Aristoflex™ et décrits notamment dans les brevets européens EP 816 403, EP 1 116 733 et EP 1 069 142. Ces épaississants synthétiques sous forme de poudre sont obtenus par polymérisation précipitante ; le (ou les) monomère(s) est (ou sont) mis en solution dans un solvant organique type benzène, acétate d'éthyle, cyclohexane, tertio-butanol ; ce procédé nécessite donc de nombreuses étapes successives de purification du produit final, pour éliminer toute trace de solvant résiduel.

Les industries de la cosmétique et de la pharmacie utilisent aussi très largement des épaississants se présentant sous la forme de latex inverses et notamment ceux commercialisés par la demanderesse. On citera par exemple, les épaississants Sepigel™ 305, Simulgel™ 600, Simulgel™ EG, Simulgel™ EPG, Simulgel™ NS, Simulgel™ A, Sepiplus™ 400, Sepiplus™ 250 et Sepiplus™ 265. Ces épaississants sont obtenus par polymérisation radicalaire en émulsion inverse. Ils présentent l'avantage d'être plus aisément manipulables, notamment à température ambiante, et se dispersent très rapidement dans l'eau. De plus, ces produits développent des performances épaississantes remarquablement élevées ; ces performances sont probablement la conséquence du procédé mis en oeuvre pour leur préparation, une réaction de polymérisation radicalaire en phase dispersée, qui conduit à des polymères de très hauts poids moléculaires.

Néanmoins, ces épaississants synthétiques se présentant sous la forme de latex inverse contiennent une huile, et un ou plusieurs agents tensioactifs qui peuvent parfois induire des réactions d'intolérance cutanée sur des sujets particulièrement sensibles ; de plus cette présence d'huile les rend inutilisables pour la préparation de gels aqueux clairs.

La demanderesse a donc développé des épaississants synthétiques ayant les performances épaississantes équivalentes ou supérieures aux latex inverses, mais mieux tolérés par la peau, en particulier du fait de l'absence de toute phase huile pouvant conduire à des gels aqueux plus clairs. Ces produits se présentent sous la forme de poudre mais possèdent des temps de dissolution, et donc une facilité de mise en oeuvre, comparable à ceux des produits se présentant sous la forme de liquides. Ces composés, décrits dans la demande de brevet européen publiée sous le numéro EP 1 496 081, sont obtenus par les techniques classiques de polymérisation, telles que la polymérisation radicalaire en phase dispersée, la polymérisation radicalaire en suspension inverse, la polymérisation radicalaire en émulsion inverse ou en microémulsion inverse. Les systèmes épaississants synthétiques obtenus sont ensuite extraits et purifiés par différentes techniques telles que la précipitation dans un tiers solvant, la précipitation dans un tiers solvant suivie éventuellement d'un lavage, d'un séchage par atomisation ou par déshydratation azéotropique, suivie éventuellement d'un lavage par un solvant judicieusement choisi. Ces épaississants synthétiques combinent donc une partie des avantages des épaississants synthétiques se présentant sous la forme de poudres classiques (absence d'huile, obtention de gels aqueux plus clairs) et les avantages des épaississants synthétiques se présentant sous la forme de latex inverse (haute vitesse de dissolution, pouvoir épaississant et propriétés stabilisantes remarquables). Cependant pour certaines utilisations, les clients utilisateurs de tels systèmes épaississants synthétiques souhaitent pouvoir fabriquer des gels encore plus clairs, que ceux obtenus aujourd'hui, voir des gels transparents. De plus, les gels obtenus avec ces épaississants synthétiques n'ont pas une stabilité satisfaisante lorsque la composition est riche en électrolytes comme c'est souvent le cas des compositions comprenant des filtres solaires et/ou des pigments colorés et/ou des extraits de végétaux riches en électrolytes.

La demanderesse a aussi développé des systèmes épaississants synthétiques comme ceux décrits dans la demande brevet français publiée sous le numéro 2 910 899, qui divulgue un terpolymère linéaire, branché ou réticulé d'au moins un monomère possédant une fonction acide fort, libre, partiellement salifiée ou totalement salifiée, avec au moins un monomère neutre, et au moins un monomère de formule (A) : 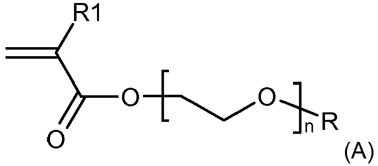 dans laquelle R1 représente un atome d'hydrogène ou un radical méthyle, R représente un radical alkyle linéaire ou ramifié comportant de huit à trente atomes de carbone et n représente un nombre supérieur ou égal à un et inférieur ou égal à cinquante. Ces polymères présentent des propriétés épaississantes très prononcées, notamment en présence d'électrolytes. Ils fonctionnent sur une large gamme de pH et permettent de réaliser des gels transparents. Cependant, les formulations à bas pH épaissies par certains d'entre eux n'ont pas une tenue aux sels satisfaisante sur le long terme et certaines d'entres elles, qui contiennent des alcools gras, ont un aspect élastique peu engageant et donnant des sensations de collant au toucher et/ou un aspect d'une crème ou d'une émulsion granuleuse et non continues.

La demanderesse a mis en évidence que ces inconvénients pouvaient être évités en sélectionnant certains de ces terpolymères, qui n'avaient pas été divulgués dans la demande de brevet français publiée sous le numéro 2 910 899, et a développé de nouveaux polyélectrolytes anioniques branchés ou réticulés, comme ceux décrits dans la demande internationale publiée sous le numéro WO 2011/030044, qui sont issus de la polymérisation radicalaire d'au moins un monomère possédant une fonction acide fort, partiellement salifiée ou totalement salifiée, avec au moins un monomère neutre, et au moins un monomère de formule (B) : 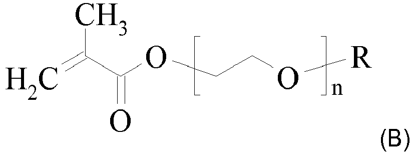 dans laquelle R représente un radical alkyle linéaire ou ramifié comportant de huit à vingt atomes de carbone et n représente un nombre supérieur ou égal à un et inférieur ou égal à trente.

Les industries de la cosmétique et de la pharmacie sont également à la recherche de formes galéniques réduisant les risques d'intolérances cutanées, et par conséquent tendent à sélectionner des ingrédients constitutifs desdites formes galéniques qui soient bien tolérés et également à diminuer, dans leur constitution, la part d'ingrédients susceptibles d'augmenter la probabilité de réactions d'intolérances sur la peau. A cet égard, les industries de la cosmétique et de la pharmacie cherchent à mettre au point des émulsions huile-dans-eau qui soient dépourvues de systèmes stabilisants comprenant des tensio-actifs émulsionnants. Les terpolymères décrits dans la demande internationale publiée sous le numéro WO 2011/030044 constituent donc des candidats idéals pour préparer des émulsions huile-dans-eau exemptes de tensio-actifs émulsionnants.

Cependant, lorsque des émulsions huile-dans-eau sont préparées par la mise en oeuvre de tels terpolymères épaississants synthétiques en absence de tensio-actifs émulsionnants, on observe l'apparition d'amas dans lesdites émulsions huile-dans-eau exemptes de tensio-actifs émulsionnants lors de leur stockage au cours du temps. Il est donc nécessaire de développer de nouvelles émulsions huile-dans-eau, exemptes de tensio-actifs émulsionnants, qui ne présentent pas lors de leur stockage prolongé d'amas au stockage, mais qui conservent une viscosité élevée en présence de milieux riches en électrolytes et sur une large gamme de pH, ainsi que des propriétés sensorielles satisfaisantes, à savoir exempte de caractère collant et filant lors de leur préhension et après application sur la peau.

Les polysaccharides sont utilisés depuis de nombreuses années comme agents modificateurs de la texture et/ou de la rhéologie pour la préparation de compositions alimentaires, cosmétiques ou pharmaceutiques. Selon leur constitution chimique, ils peuvent être utilisés comme agents gélifiants et/ou comme agents épaississants. Par agent épaississant, on entend un composé chimique qui augmente la viscosité du milieu dans lequel il est introduit. Par agent gélifiant, on entend un composé qui transforme un milieu liquide en un état structuré, qui ne coule pas, par formation d'un réseau tridimensionnel au sein du liquide ; le gel étant considéré comme un état intermédiaire entre l'état liquide et l'état solide.

Les polysaccharides sont des polymères de saccharides. La définition IUPAC des saccharides désigne des oses, des composés d'oses proprement dits et leurs dérivés obtenus soit par réduction d'un groupement carbonyle, soit par oxydation d'une ou plusieurs fonctions hydroxyles, soit par le remplacement d'une ou plusieurs fonctions hydroxyles par un atome d'hydrogène, par un groupement amine, une fonction phosphate, une fonction sulfate.

Les polysaccharides les plus couramment utilisés pour la préparation de compositions alimentaires, cosmétiques ou pharmaceutiques, sont majoritairement constitués d'oses, tels que le glucose, le galactose, le mannose ou de dérivés d'oses pour lesquels la fonction hydroxyle du carbone terminal a été oxydée en fonction carboxyle. Par conséquent, on peut distinguer parmi les polysaccharides deux groupes distincts : les polysaccharides constitués uniquement d'oses (ou poly-oses) et les polysaccharides constitués de dérivés d'oses. Parmi les polysaccharides composés uniquement d'oses, on peut distinguer :
- les glucanes, qui sont des homopolymères de glucose très abondants dans la nature,
- les glucomannoglycanes,
- les xyloglycanes,
- les galactomannanes, qui sont des polymères dont la chaîne principale est constituée d'unités de D-mannose, reliées entre elles en β-1,4, et sur laquelle des unités de D-galactose sont greffées latéralement par des liaisons α-1,6.

Les galactomannanes se rencontrent dans plusieurs espèces végétales, et plus particulièrement dans les espèces légumineuses dans lesquelles ils constituent l'albumen des graines.

En fonction de l'origine végétale de la provenance des galactomannanes, le degré de substitution (DS) des unités de D-galactose sur la chaîne principale de D-mannose varie entre 0 et 1 :
- les galactomannanes provenant de la gomme de cassia présentent un degré de substitution (DS) d'environ 1/5, signifiant le greffage latéral d'une unité de D-galactose toutes les 5 unités de D-mannose présentes sur la chaîne principale du polysaccharide,
- les galactomannanes provenant de la gomme de caroube présentent un degré de substitution (DS) d'environ 1/4, signifiant le greffage latéral d'une unité de D-galactose toutes les 4 unités de D-mannose présentes sur la chaîne principale du polysaccharide,
- les galactomannanes provenant de la gomme de tara présentent un degré de substitution (DS) d'environ 1/3, signifiant le greffage latéral d'une unité de D-galactose toutes les 3 unités de D-mannose présentes sur la chaîne principale du polysaccharide,
- les galactomannanes provenant de la gomme de guar présentent un degré de substitution (DS) d'environ 1/2, signifiant le greffage latéral d'une unité de D-galactose toutes les 2 unités de D-mannose présentes sur la chaîne principale du polysaccharide,
- les galactomannanes provenant de la gomme de fenugrec présentent un degré de substitution (DS) d'environ 1/1, signifiant le greffage latéral d'une unité de D-galactose pour quasiment toutes les unités de D-mannose présentes sur la chaîne principale du polysaccharide.

En tant que sous-catégorie des polysaccharides, les galactomannanes ont déjà été associés à des épaississants synthétiques résultant de la polymérisation radicalaire de monomères tels que l'acide acrylique, les esters de l'acide acrylique, l'acide 2-acrylamido-2-méthyl-propanesulfonique et/ou de ses sels, l'acrylamide, l'acrylate de 2-hydroxy éthyle.

Le brevet américain publié sous le numéro 4,540,510 décrit la préparation de gels aqueux mettant en oeuvre des homopolymères de l'acide 2-acrylamido-2-méthyl-propanesulfonique et des galactomannanes, comme ceux issus de la gomme de guar et de la gomme de tara. Cependant, les gels aqueux qui sont obtenus et utilisés pour des applications dans le domaine de la fracturation des cavités souterraines, ou de la préparation de pâtes pigmentaires pour des impressions sur du textile, ou de la suspension de pigment dans des peintures, ou dans la préparation de formulations cosmétiques comprenant des alcools comme co-solvants, ne permettent pas d'atteindre des niveaux de viscosité suffisants pour permettre la préparation d'émulsions huile-dans-eau riches en électrolytes et stables au stockage.

La demande de brevet français publiée sous le numéro 2 940 111 décrit l'utilisation des compositions comprenant des polysaccharides, pouvant être associés à des gélifiants hydrophiles notamment choisis parmi des copolymères comportant l'acide 2-acrylamido-2-méthyl-propanesulfonique et l'acrylamide comme monomères constitutifs, ou des comportant l'acide 2-acrylamido-2-méthyl propanesulfonique et méthacrylates d'alkyles polyoxyéthylénés. Ces compositions sont destinées à des utilisations de maquillage, possédant la propriété de ne pas transférer sur les supports sur lesquelles elles sont mises en contact ainsi que la propriété consistant à résister à l'eau après application sur la peau. Cependant, les gélifiants hydrophiles décrits dans la demande de brevet Français publiée sous le numéro 2 940 111 sont connus pour ne pas permettre d'atteindre des niveaux élevés de viscosité en présence de milieux riches en électrolytes.

Les inventeurs ont donc cherché à développer de nouvelles émulsions huile-dans-eau, exemptes de tensio-actifs émulsionnants dans leur système stabilisant, riches en sels, conservant une viscosité élevée et un aspect homogène après une période de stockage prolongée.

C'est pourquoi selon un premier aspect, l'invention a pour objet une composition (C₁) se présentant sous la forme d'une émulsion de type huile-dans-eau, caractérisée en ce qu'elle comprend pour 100% de sa masse :
- de 5% à 55% massique, plus particulièrement de 7% à 30% massique et encore plus particulièrement de 10% à 20% massique d'une phase grasse (P₁) constituée d'au moins une huile et/ou d'au moins une cire,
- de 0,025% à 3,75% massique, , plus particulièrement de 0,125% à 3%, et encore plus particulièrement de 0,125% à 2,25% d'au moins un polyélectrolyte anionique réticulé (PA) issu de la polymérisation d'au moins un monomère possédant une fonction acide fort, partiellement ou totalement salifiée, avec au moins un monomère neutre, et au moins un monomère de formule (I) : 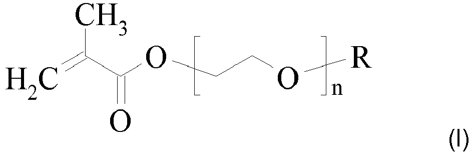 dans laquelle R représente un radical alkyle linéaire ou ramifié comportant de huit à vingt atomes de carbone et n représente un nombre supérieur ou égal à un et inférieur ou égal à vingt, en présence d'au moins un agent de réticulation,
   - de 0,025% à 3,75% massique, plus particulièrement de 0,125% à 3% massique et encore plus particulièrement de 0,125% à 2,25% massique d'au moins un galactomannane (GM) présentant un degré de substitution (DS) d'environ 1/1 ou d'environ 1/2 ou d'environ 1/4 ou d'environ 1/5,
   - de 37,5% à 94,95% massique, plus particulièrement de 55% à 94,95% massique, et encore plus particulièrement de 65% à 94,95% massique d'une phase aqueuse (P₂) cosmétiquement acceptable, ladite phase aqueuse (P₂) comprenant pour 100% de sa masse de 1% à 25% massique plus particulièrement de 1,5% à 20% massique, et encore plus particulièrement de 2% à 10% massique d'au moins un sel (S) se présentant sous une forme dissoute,
ladite composition (C₁) étant en outre caractérisée en ce que le rapport massique entre le galactomannane (GM) et le polyélectrolyte anionique réticulé (PA) est supérieur ou égal à 1/3 et inférieur ou égal à 3/1, plus particulièrement supérieur ou égal à 1/2 et inférieur ou égal à 3/2, et encore plus particulièrement supérieur ou égal à 2/3 et inférieur ou égal à 1.

Par « huiles », on entend dans la présente demande les composés et/ou les mélanges de composés insolubles dans l'eau, se présentant sous un aspect liquide à une température de 25°C. Parmi les huiles utilisables dans la phase grasse (P₁) de la composition (C₁) objet de la présente invention, on peut citer :
- les huiles minérales telles que l'huile de paraffine, l'huile de vaseline, les isoparaffines ou les huiles blanches minérales ;
- les huiles d'origine animale, telles que le squalène ou le squalane ;
- les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ;
- les huiles végétales éthoxylées ;
- les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées ;et
- les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Par « cires », on entend dans la présente demande les composés et/ou les mélanges de composés insolubles dans l'eau, se présentant sous un aspect solide à une température supérieure ou égale à 45°C. Parmi les cires utilisables dans la phase grasse (P₁) de la composition (C₁) objet de la présente invention, on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante.

Parmi les autres matières grasses que l'on peut associer à la phase grasse (P₁) de la composition (C₁) objet de la présente invention, on peut citer les alcools gras saturés ou insaturés, linéaires ou ramifiés, ou les acides gras saturés ou insaturés, linéaires ou ramifiés.

Dans la composition (C₁) objet de la présente invention, par « un galactomannane (GM) présentant un degré de substitution (DS) d'environ 1/5 », on désigne un polysaccharide dont la chaîne principale est constituée d'unités de D-mannose, reliées entre elles en position β-1,4, et sur laquelle des unités de D-galactose sont greffées latéralement par des liaisons α-1,6, de façon à ce que le greffage d'une unité de D-galactose s'observe en moyenne toutes les 5 unités de D-mannose présentes sur la chaîne principale du polysaccharide. Le galactomannane (GM) tel que défini précédemment provient plus particulièrement de la gomme de cassia.

Dans la composition (C₁) objet de la présente invention, par « un galactomannane (GM) présentant un degré de substitution (DS) d'environ 1/4 », on désigne un polysaccharide dont la chaîne principale est constituée d'unités de D-mannose, reliées entre elles en position β-1,4, et sur laquelle des unités de D-galactose sont greffées latéralement par des liaisons α-1,6, de façon à ce que le greffage d'une unité de D-galactose s'observe en moyenne toutes les 4 unités de D-mannose présentes sur la chaîne principale du polysaccharide. Le galactomannane (GM) tel que défini précédemment provient plus particulièrement de la gomme de caroube.

Dans la composition (C₁) objet de la présente invention, par « un galactomannane (GM) présentant un degré de substitution (DS) d'environ 1/2 », on désigne un polysaccharide dont la chaîne principale est constituée d'unités de D-mannose, reliées entre elles en position β-1,4, et sur laquelle des unités de D-galactose sont greffées latéralement par des liaisons α-1,6, de façon à ce que le greffage d'une unité de D-galactose s'observe en moyenne toutes les 2 unités de D-mannose présentes sur la chaîne principale du polysaccharide. Le galactomannane (GM) tel que défini précédemment provient plus particulièrement de la gomme de guar.

Dans la composition (C₁) objet de la présente invention, par « un galactomannane (GM) présentant un degré de substitution (DS) d'environ 1/1 », on désigne un polysaccharide dont la chaîne principale est constituée d'unités de D-mannose, reliées entre elles en position β-1,4, et sur laquelle des unités de D-galactose sont greffées latéralement par des liaisons α-1,6, de façon à ce que le greffage d'une unité de D-galactose s'observe en moyenne toutes les unités de D-mannose présentes sur la chaîne principale du polysaccharide. Le galactomannane (GM) tel que défini précédemment provient plus particulièrement de la gomme de fenugreck.

Selon un aspect particulier de la présente invention, la composition (C₁) telle que définie précédemment est caractérisée en ce que ledit galactomannane (GM) présente un degré de substitution (DS) d'environ 1/2.

Par polyélectrolyte anionique réticulé (PA), on désigne dans la définition de la composition (C₁) objet de la présente invention, un polyélectrolyte anionique réticulé non linéaire, se présentant à l'état de réseau tridimensionnel insoluble dans l'eau, mais gonflable à l'eau et conduisant à l'obtention d'un gel chimique.

Par partiellement salifié ou totalement salifié, on signifie dans la définition du polyélectrolyte anionique réticulé (PA) présent de la composition (C₁) telle que définie ci-dessus, que ladite fonction acide fort du monomère en comportant est, partiellement ou totalement salifiée, généralement sous forme de sel de métal alcalin, comme par exemple le sel de sodium ou le sel de potassium, ou sous forme de sel d'ammonium.

Dans la composition (C₁) telle que définie ci-dessus et objet de la présente invention, ledit polyélectrolyte anionique réticulé (PA) tel que défini précédemment comporte généralement entre 5% molaire et 95% molaire de monomères à faonction acide fort, plus particulièrement entre 10% molaire et 90% molaire, et tout particulièrement entre 20% molaire et 80% molaire.

Dans la composition (C₁) telle que définie ci-dessus et objet de la présente invention, ledit polyélectrolyte anionique réticulé (PA) tel que défini précédemment comporte généralement entre 4,9% molaire et 90% molaire de monomère neutre, plus particulièrement entre 9,5% molaire et 85% molaire, et tout particulièrement entre 15% molaire et 75% molaire.

Dans la composition (C₁) telle que définie ci-dessus et objet de la présente invention, ledit polyélectrolyte anionique réticulé (PA) tel que défini précédemment, comporte généralement entre 0,1% molaire et 10% molaire de monomères de formule (I) et plus particulièrement entre 0,5% molaire et 5% molaire.

Dans la composition (C₁) telle que définie ci-dessus et objet de la présente invention, dans ledit polyélectrolyte anionique réticulé (PA) tel que défini ci-dessus, la fonction acide fort dudit monomère en comportant est notamment la fonction acide sulfonique, partiellement ou totalement salifiée.

Dans la composition (C₁) telle que définie ci-dessus et objet de la présente invention, dans ledit polyélectrolyte anionique réticulé (PA) tel que défini ci-dessus, le monomère neutre est notamment choisi parmi l'acrylamide, le méthacrylamide, les N-alkyl acrylamide, dans lesquels le groupe alkyle comporte de un à quatre atomes de carbone, comme par exemple le N-méthyl acrylamide, le N-éthyl acrylamide, le N-propyl acrylamide, le N-isopropyl acrylamide, le N-butyl acrylamide, le N-(tert-butyl) acrylamide, les N-alkyl méthacrylamide, dans lesquels le groupe alkyle comporte de un à quatre atomes de carbone, comme par exemple le N-méthyl méthacrylamide, le N-éthyl méthacrylamide, le N-propyl méthacrylamide, le N-isopropyl méthacrylamide, N-butyl méthacrylamide ou le N-(tert-butyl)méthacrylamide, les N,N-dialkyl acrylamide, dans lesquels chacun des groupes alkyle comportent entre un et quatre atomes de carbones, comme par exemple, le N,N-diméthyl acrylamide, le N,N-diéthyl acrylamide, le N,N-dipropyl acrylamide, l'acrylate de (2-hydroxy éthyle), l'acrylate de (2,3-dihydroxy propyle), le méthacrylate de (2-hydroxy éthyle), le méthacrylate de (2,3-dihydroxy propyle), le diacétone acrylamide ou un dérive éthoxylé de poids moléculaire compris entre 400 g/mol et 1000 g/mol de chacun de ces esters, ou le vinylpyrrolidone.

Dans la formule (I) telle que définie ci-dessus et objet de la présente invention, par radical alkyle linéaire ou ramifié comportant de huit à vingt atomes de carbone, on désigne plus particulièrement pour R :
- ou bien un radical dérivé des alcools primaires linéaires tels que par exemple, le radical octyle, décyle, undécyle, dodécyle, tridécyle, tétradécyle, pentadécyle, hexadécyle, heptadécyle, octadécyle, nonadécyle ou eicosyle ;
- ou bien un radical dérivé des alcools de Guerbet, qui sont des 1-alcanols ramifiés répondant à la formule générale :

   CH₃-(CH₂)ₚ-CH[CH₃-(CH₂)ₚ₋₂]-CH₂OH,

   dans laquelle p représente un nombre entier compris entre 2 et 9, tels que, par exemple, les radicaux 2-éthyl hexyle, 2-propyl heptyle, 2-butyl octyle, 2-pentyl nonyle, 2-hexyl décyle ou 2-octyl dodécyle ;
- ou bien un radical dérivé des isoalcanols répondant à la formule générale :

   CH₃-CH(CH₃)-(CH₂)ₘ-CH₂OH,

   dans laquelle m représente un nombre entier compris entre 2 et 16, tels que, par exemple, les radicaux 4-méthyl pentyle, 5-méthyl hexyle, 6-méthyl heptyle, 15-méthyl pendadécyle ou 16-méthyl heptadécyle, soit les radicaux 2-hexyl octyle, 2-octyl décyle ou 2-hexyl dodécyle.

Selon un aspect particulier, l'invention a pour objet une composition (C₁) telle que définie précédemment, caractérisée en ce que ledit polyélectrolyte anionique réticulé (PA) comporte pour 100% molaire de ses monomères constitutifs :
- de 20% molaire à 80% molaire d'unités monomériques issues d'un monomère comportant une fonction acide fort partiellement ou totalement salifiée ;
- de 15% molaire à 75% molaire d'unités monomériques issues d'un monomère neutre ;
- de 0,5% à 5% molaire d'unités monomériques issues d'un monomère de formule (I).

Selon un autre aspect particulier, l'invention a pour objet une composition (C₁) telle que définie précédemment caractérisée en ce que dans ledit polyélectrolyte anionique réticulé (PA) ledit monomère possédant une fonction acide fort est l'acide 2-méthyl 2-[(1-oxo 2-propènyl) amino] 1-propanesulfonique partiellement salifié ou totalement salifié., et plus particulièrement l'acide 2-méthyl 2-[(1-oxo 2-propènyl) amino] 1 propanesulfonique partiellement salifié ou totalement salifié sous forme de sel de métal alcalin, comme par exemple le sel de sodium ou le sel de potassium, ou sous forme de sel d'ammonium.

Selon un autre aspect particulier, l'invention a pour objet une composition (C₁) telle que définie précédemment caractérisée en ce que dans ledit polyélectrolyte anionique réticulé (PA), ledit monomère neutre est choisi parmi l'acrylamide, l'acrylate de (2-hydroxy éthyle) ou le N,N-diméthyl acrylamide.

Selon un aspect particulier de la présente invention, celle-ci a pour objet une composition (C₁) telle que définie précédemment, caractérisée en ce que dans ledit polyélectrolyte anionique réticulé (PA) et pour ledit monomère de formule (I) telle que définie précédemment, R représente un radical alkyle comportant de 12 à 18 atomes de carbone.

Selon un autre aspect particulier, l'invention a pour objet une composition (C₁) telle que définie précédemment, caractérisée en ce que dans ledit polyélectrolyte anionique réticulé (PA) et pour ledit monomère de formule (I) telle que définie précédemment, n représente un nombre entier compris entre 3 et 20.

Selon un aspect encore plus particulier, l'invention a pour objet une composition (C₁) telle que définie précédemment, caractérisée en ce que dans ledit polyélectrolyte anionique réticulé (PA), ledit monomère de formule (I) est le méthacrylate de lauryle tétraéthoxylé.

Selon un aspect encore plus particulier, l'invention a pour objet une composition (C₁) telle que définie précédemment, caractérisée en ce que le monomère de formule (I) compris dans le polyélectrolyte anionique réticulé (PA) est le méthacrylate de stéaryle eicosaéthoxylé.

Selon un aspect encore plus particulier, l'invention a pour objet une composition (C₁) telle que définie précédemment, caractérisée en ce que le monomère de formule (I) compris dans le polyélectrolyte anionique réticulé (PA) est le méthacrylate de béhényle éthoxylé à 25 moles d'oxyde d'éthylène.

Selon un autre aspect particulier, l'invention a pour objet une composition (C₁) telle que définie précédemment, pour laquelle ledit polyélectrolyte anionique réticulé (PA) est réticulé avec un composé diéthylénique ou polyéthylènique dans la proportion molaire exprimée par rapport aux monomères mis en oeuvre, de 0,005% à 1%, plus particulièrement de 0,01% à 0,5% et tout particulièrement de 0,01% à 0,25%. L'agent de réticulation est plus particulièrement choisi parmi le diméthacrylate d'éthylèneglycol, le tétraallyloxyéthane, le diacrylate d'éthylèneglycol, le diallyl urée, le triallyl amine, le triméthylol propanetriacrylate ou le méthylène-bis(acrylamide) ou un mélange de ces composés.

Le polyélectrolyte anionique réticulé (PA) mis en oeuvre dans la composition (C₁) telle que définie précédemment et objet de la présente invention peut également comprendre divers additifs, tels que des agents complexants, des agents de transfert ou des agents limiteurs de chaîne.

Selon un aspect particulier, l'invention a pour objet une composition (C₁) telle que décrite précédemment dans laquelle ledit polyélectrolyte anionique réticulé (PA) est choisi parmi les terpolymères de l'acide 2-méthyl 2-[(1-oxo 2-propènyl) amino] 1-propanesulfonique partiellement salifié sous forme de sel d'ammonium, de l'acrylamide et du méthacrylate de lauryle tétraéthoxylé, réticulé au triméthylol propanetriacrylate, les terpolymères de l'acide 2- méthyl 2-[(1-oxo 2-propènyl) amino] 1-propanesulfonique partiellement salifié sous forme de sel d'ammonium, de l'acrylamide et du méthacrylate de stéaryle d'eicosaéthoxylé, réticulé au triméthylol propanetriacrylate, les terpolymères de l'acide 2-méthyl 2-[(1-oxo 2-propènyl) amino] 1-propanesulfonique partiellement salifié sous forme de sel d'ammonium, de l'acrylate de 2-hydroxy éthyle et du méthacrylate de lauryle tétraéthoxylé, réticulé au triméthylol propanetriacrylate, les terpolymères de l'acide 2-5 méthyl 2-[(1-oxo 2-propènyl) amino] 1-propanesulfonique partiellement salifié sous forme de sel d'ammonium, de l'acrylate de 2-hydroxy éthyle et du méthacrylate de stéaryle d'eicosaéthoxylé, réticulé au triméthylol propanetriacrylate, les terpolymères de l'acide 2-méthyl 2-[(1-oxo 2-propènyl) amino] 1-propanesulfonique partiellement salifié sous forme de d'ammonium, du N,N-diméthyl acrylamide et du méthacrylate de lauryle tétraéthoxylé, réticulé au triméthylol propanetriacrylate ou les terpolymères de l'acide 2-méthyl 2-[(1-oxo 2-propènyl) amino] 1-propanesulfonique partiellement salifié sous forme de sel d'ammonium, du N,N-diméthyl acrylamide et du méthacrylate de stéaryle d'eicosaéthoxylé, réticulé au triméthylol propanetriacrylate.

Selon un aspect encore plus particulier, l'invention a pour objet une composition (C₁) telle que décrite précédemment, caractérisée en ce que ledit polyélectrolyte anionique réticulé (PA) est un terpolymère de l'acide 2-méthyl 2- [(1-oxo 2-propènyl) amino] 1-propanesulfonique partiellement salifié sous forme de sel d'ammonium, du N,N-diméthyl acrylamide et du méthacrylate de lauryle tétraéthoxylé, réticulé au triméthylol propanetriacrylate.

Dans la composition (C₁) selon l'invention, la combinaison in situ du polyélectrolyte anionique réticulé (PA) et du galactomannane (GM) tels que définis précédemment et dans les proportions telles que définies précédemment, constitue le système stabilisant de ladite composition (C₁).

L'expression « cosmétiquement acceptable » utilisée dans la définition de la phase aqueuse (P₂) de la composition (C₁) objet de la présente invention, signifie selon la directive du Conseil de la Communauté Economique Européenne N°76/768/CEE du 27 juillet 1976 modifiée par la directive N°93/35/CEE du 14 juin 1993, que ladite phase aqueuse (P₂) comprend de l'eau et toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Une phase aqueuse (P₂) cosmétiquement acceptable comprise dans la composition (C₁) objet de la présente invention contient de l'eau, et peut contenir classiquement un ou plusieurs solvants organiques cosmétiquement acceptables, un mélange d'eau et d'un ou plusieurs solvants organiques cosmétiquement acceptables. Les solvants cosmétiquement acceptables peuvent plus particulièrement être choisis parmi les alcools polyhydriques comme par exemple le glycérol, le diglycérol, le triglycérol, les oligomères du glycérol, le xylitol, l'érythritol, le sorbitol, le méthyl-2,-propanediol-1,3; les alcools polyhydriques alcoxylés ; les glycols, comme par exemple le butylène glycol, l'hexylène glycol, le caprylyl glycol ou 1,2 octanediol ou 1,2 pentanediol, le pentylène glycol, le monopropylène glycol, le dipropylène glycol, l'isoprène glycol, le butyldiglycol, les polyéthylènes glycols dont le poids moléculaire est compris entre 200g.mol-1 et 8000g.mol-1 ; ou les alcools hydrosolubles comme par exemple l'éthanol, l'isopropanol ou le butanol.

Dans la composition (C₁) telle que définie précédemment et objet de la présente invention, par sel (S) on désigne un composé hétéropolaire dont le réseau cristallin comprend la participation d'au moins un type de cation différent des ions hydrogène et d'au moins un type d'anion différent des ions hydroxydes.

Selon un aspect particulier, le sel (S) se présentant sous une forme dissoute dans la phase aqueuse (P₂) de la composition (C₁) objet de la présente invention, est sélectionné parmi les sels inorganiques et parmi les sels organiques.

Selon cet aspect particulier, le sel (S) est particulièrement sélectionné parmi les sels inorganiques.

Selon un plus aspect particulier, l'invention a pour objet une composition (C₁) telle que définie précédemment, caractérisée en ce que le sel (S) est un sel inorganique constitué par un cation qui est l'ion ammonium ou un cation métallique et par un anion sélectionné parmi les éléments du groupe constitué par les halogénures, les carbonates, les bicarbonates , les phosphates, les nitrates, les borates et les sulfates.

Selon un aspect plus particulier, l'invention a pour objet une composition (C₁) telle que définie précédemment caractérisée en ce que le sel (S) est un sel inorganique dont le cation métallique est un cation monovalent ou multivalent choisi parmi les éléments du groupe constitué par les cations du sodium, du potassium, du lithium, du calcium, du magnésium, du zinc, du manganèse, du fer, du cuivre, du cobalt, de l'argent, de l'or, de l'aluminium, du barium, du bismuth, du sélénium, du zirconium, du strontium et de l'étain.

Selon un aspect encore plus particulier, l'invention a pour objet une composition (C1) telle que définie précédemment caractérisée en ce que le sel (S) est un sel inorganique choisi parmi les éléments du groupe constitué par le chlorure de sodium, le chlorure de calcium, le chlorure de magnésium, le sulfate de calcium, le sulfate d'ammonium, le carbonate de calcium, le sulfate de zinc, le sulfate de magnésium, le borate de sodium.

Selon un autre aspect particulier, le sel (S) est particulièrement sélectionné parmi les sels organiques.

Selon un aspect particulier, l'invention a pour objet une composition (C₁) telle que définie précédemment, caractérisée en ce que le sel (S) est un sel organique constitué par un cation qui est l'ion ammonium ou un cation métallique et par un anion organique qui est un composé organique possédant au moins une fonction acide carboxylique sous forme carboxylate ou au moins une fonction acide sulfonique sous forme sulfonate ou au moins une fonction sulphate .

Selon cet aspect particulier, l'invention a pour objet une composition (C₁) telle que définie précédemment caractérisée en ce que le sel (S) est un sel organique constitué par un cation métallique monovalent ou multivalent plus particulièrement choisi parmi les éléments du groupe constitué par les cations du sodium, du potassium, du lithium, du calcium, du magnésium, du zinc, du manganèse, du fer, du cuivre, du cobalt de l'argent, de l'or, de l'aluminium, du barium, du bismuth, du sélénium, du zirconium, du strontium et de l'étain. Selon cet aspect particulier, le sel (S) est un sel organique constitué par le cation choisi parmi les éléments groupe constitué par les cations du sodium, du calcium, du magnésium, du zinc et du manganèse, et encore plus particulièrement le sel (S) est un sel organique constitué par le cation du sodium.

Selon un aspect particulier, l'invention a pour objet une composition (C₁) telle que définie précédemment caractérisée en ce que le sel (S) est un sel organique constitué par un cation qui est l'ion ammonium ou un cation métallique tel que décrit précédemment, et par un anion organique qui est un composé organique possédant au moins une fonction acide carboxylique sous forme carboxylate choisi parmi les éléments du groupe constitué par l'acide glycolique, l'acide citrique, l'acide tartrique, l'acide salicylique, l'acide lactique, l'acide mandélique, l'acide ascorbique, l'acide pyruvique, l'acide fumarique, l'acide rétinoïque, l'acide benzoïque, l'acide kojique, l'acide malique, l'acide gluconique, l'acide galacturonique, l'acide proprionique, l'acide heptanoïque, l'acide 4-amino benzoïque, l'acide cinnamique, l'acide benzalmalonique, l'acide aspartique et l'acide glutamique.

Selon un aspect encore plus particulier, l'invention a pour objet une composition (C₁) telle que définie précédemment, caractérisée en ce que le sel (S) est un sel organique sélectionné parmi les éléments du groupe constitué par le glycolate de sodium, le citrate de sodium, le salicylate de sodium, le lactate de sodium, le gluconate de sodium, le gluconate de zinc, le gluconate de manganèse, le gluconate de cuivre et l'aspartate de magnésium.

Selon un autre aspect particulier, l'invention a pour objet une composition (C₁) telle que définie précédemment caractérisée en ce que le sel (S) est un sel organique constitué par un cation qui est l'ion ammonium ou un cation métallique tel que décrit précédemment, et par un anion organique qui est un composé organique possédant au moins une fonction acide sulfonique sous forme sulfonate choisi parmi les éléments du groupe constitué par l'acide 2-phényl benzimidazole-5-sulphonique, les acides sulphoniques dérivés des benzophénones, comme par exemple l'acide 4-hydroxy 2-méthoxy 5-(oxo-phénylméthyl) benzènesulphonique (ledit acide étant enregistré sous l'appellation Benzophénone-4), les acides sulphoniques dérivés du 3-benzylidene camphre comme par exemple l'acide 4-(2-oxo 3-bornylidenemethyl) benzènesulphonique ou l'acide 2-méthyl 5-(2-oxo-3-bornylideneméthyl) benzènesulphonique.

Selon un aspect encore plus particulier, l'invention a pour objet une composition (C₁) telle que définie précédemment, caractérisée en ce que le sel (S) est un sel organique sélectionné parmi les éléments du groupe constitué par le 2-phénylbenzimidazole-5-sulphonate de sodium et le 4-hydroxy 2-méthoxy 5-(oxo-phénylméthyl) benzènesulphonate de sodium.

L'acide_2-phényl benzimidazole-5-sulphonique est commercialisé notamment sous le nom de marque EUSOLEX™232 par la société Merck. Le 4-hydroxy 2-méthoxy 5-(oxophénylméthyl) benzènesulphonate de sodium est enregistré sous l'appellation benzophénone-5.

De façon générale, la composition (C₁) objet de la présente invention comporte, en plus de ladite phase grasse (P₁), du système stabilisant composé de la combinaison *in situ* du polyélectrolyte anionique réticulé (PA) et du galactomannane (GM) tels que définis précédemment, et de ladite phase aqueuse cosmétiquement acceptable (P₂) tels que définis précédemment des adjuvants et ou des additifs habituellement mis en oeuvre dans le domaine des formulations cosmétiques, dermocosmétiques, pharmaceutiques ou dermo-pharmaceutiques. Parmi les adjuvants susceptibles d'être présents dans les compositions (C₁) objets de la présente invention, on peut citer des composés filmogènes, des agents hydrotropes, des agents plastifiants, des agents opacificants, des agents nacrants, des agents surgraissants, des agents séquestrants, des agents chélatants, des agents tensioactifs détergents non-ioniques, des agents antioxydants, des parfums, des agents conservateurs, des agents conditionneurs, des agents blanchissants destinés à la décoloration des poils et de la peau, des principes actifs destinés à apporter une action traitante vis à vis de la peau ou des cheveux, des charges minérales ou des pigments, des particules procurant un effet visuel ou destinées à l'encapsulation d'actifs, des particules exfoliantes, des agents de texture, des azurants optiques, des agents répulsifs pour les insectes.

Parmi les agents opacifiants et/ou nacrants que l'on peut associer à la composition (C₁) objet de la présente invention, on citera particulièrement les palmitate, les stéarate ou les hydroxystéarates de sodium ou de magnésium, les monostéarate ou distéarate d'éthylène ou de polyéthylène glycol, les alcools gras, les homopolymères et copolymères de styrène tels que le styrène acrylate copolymère commercialisé sous l'appellation MONTOPOL™ OP1 par la société SEPPIC.

Parmi les agents de texture que l'on peut associer à la composition (C₁) objet de la présente invention, on peut citer des dérives N-acyles d'acides amines, comme la lauroyl lysine commercialisée sous l'appellation AMINOHOPE™LL par la société AJINOMOTO, l'octenyl starch succinate commercialise sous l'appellation DRYFLO™ par la société NATIONAL STARCH, le myristyl polyglucoside commercialisé par SEPPIC sous l'appellation MONTANOV™ 14, les fibres de cellulose, les fibres de coton, les fibres de chitosane, le talc, la sericite, le mica.

Parmi les principes actifs que l'on peut associer à la composition (C₁) objet de la présente invention, on peut citer par exemple les vitamines et leurs dérives, notamment leurs esters, tels que le rétinol (vitamine A) et ses esters (palmitate de rétinyle par exemple), l'acide ascorbique (vitamine C) et ses esters, les dérives de sucre de l'acide ascorbique (comme par exemple l'ascorbyl glucoside), le tocophérol (vitamine E) et ses esters (comme par exemple l'acétate de tocophérol), les vitamine B3 ou B10 (niacinamide et ses dérivés); les composés montrant une action éclaircissante ou dépigmentante de la peau comme par exemple le SEPIWHITE™MSH, l'arbutine, l'acide kojique, l'hydroquinone, le VEGEWHITE™, la GATULINE™, le SYNERLIGHT™, le BIOWHITE™, le PHYTOLIGHT™, le DERMALIGHT™, la CLARISKIN™, le MELASLOW™, le DERMAWHITE™, l'ETHIOLINE, le MELAREST™, le GIGAWHITE™, l'ALBATINE™, le LUMISKIN™ ; les composés montrant une action apaisante comme le SEPICALM™ S, l'allantoïne et le bisabolol ; les agents anti-inflammatoires ; les composés montrant une action hydratante comme par exemple l'urée, les hydroxyurées, le glycérol, les polyglycérols, l'AQUAXYL™, le glycérolglucoside ; les extraits de polyphénols comme par exemple les extraits de raisin, les extraits de pin, les extraits de vin, les extraits d'olives ; les composé montrant une action amincissante ou lipolytique comme la caféine ou ses dérivés, l'ADIPOSLIM™, l'ADIPOLESS™ ; les protéines N-acylées ; les peptides N-acylés comme par exemple le MATRIXIL™ ; les acides aminés N-acylés ; les hydrolysâts partiels de protéines N-acylés ; les acides aminés ; les peptides ; les hydrolysâts totaux de protéines ; les extraits de soja, par exemple la Raffermine™ ; les extraits de blé par exemple la TENSINE™ ou la GLIADINE™ ; les extraits végétaux, tels que les extraits végétaux riches en tanins, les extraits végétaux riches en isoflavones ou les extraits végétaux riches en terpènes ; les extraits d'algues d'eau douce ou marines ; les extraits marins en général comme les coraux ; les cires essentielles ; les extraits bactériens ; les céramides ; les phospholipides ; les composés montrant une action antimicrobienne ou une action purifiante, comme par exemple le LIPACIDE™ C8G, le LIPACIDE™ UG, le SEPICONTROL™ A5 ; l'OCTOPIROX™ ou le SENSIVA™ SC50 ; les composés montrant une propriété énergisante ou stimulante comme le Physiogényl™, le panthénol et ses dérivés comme le SEPICAP™ MP ; les actifs anti-age comme le SEPILIFT™ DPHP, le LIPACIDE™ PVB, le SEPIVINOL™, le SEPIVITAL™, le MANOLIVA™, le PHYTOAGE™, le TIMECODE™ ; le SURVICODE™ ; les actifs anti-photo vieillissement ; les actifs protecteurs de l'intégrité de la jonction dermo-épidermique ; les actifs augmentant la synthèse des composants de la matrice extracellulaire comme par exemple le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme par exemple les dérivés de l'acide nicotinique) ou des produits créant une sensation de « fraîcheur » sur la peau (comme par exemple le menthol et des dérivés) ; les actifs améliorant la microcirculation cutanée, par exemple les veinotoniques ; les actifs drainants ; les actifs à visée décongestionnante comme par exemple les extraits de ginko biloba, de lierre, de marron d'inde, de bambou, de ruscus, de petit houx, de *centalla asiatica*, de fucus, de romarin, de saule.

Parmi les principes actifs que l'on peut associer à la composition (C₁) objet de la présente invention, on peut citer plus particulièrement les agents de bronzage ou de brunissement de la peau, comme par exemple la dihydroxyacétone, l'isatinne, l'alloxane, la ninhydrine, le glycéraldéhyde, l'aldéhyde mésotartrique, la glutaraldéhyde, l'érythrulose.

Parmi les agents tensioactifs détergents non ioniques que l'on peut associer à la composition (C₁) objet de la présente invention, on peut citer les dérivés éthoxylés d'alcools gras comportant de 8 à 12 atomes de carbone, les dérivés éthoxylés d'acides gras comportant de 8 à 12 atomes de carbone, les dérivés éthoxylés d'esters gras comportant de 8 à 12 atomes de carbone, les dérivés éthoxylés de monoglycérides comportant de 8 à 12 atomes de carbone,les alkylpolyglycosides de formule (II) :

R2-O-(S)y-H (II)

dans laquelle y représente un nombre décimal compris entre 1 et 5, S représente le reste d'un sucre réducteur et R2 représente un radical alkyle linéaire ou ramifié, saturé ou insaturé, ayant de 5 à 16 atomes de carbone, de préférence de 8 à 14 atomes de carbone, ou un mélange de composés de formule (II).

Les agents tensioactifs détergents non ioniques que l'on peut associer à la composition (C₁) objet de la présente invention, sont plus particulièrement choisi parmi les éléments du groupe constitué par le caprylyl capryl glucosides, commercialisé notamment sous le nom de marque ORAMIX™CG 110 par la société SEPPIC, le décylglucoside, commercialisé notamment sous le nom de marque ORAMIX™NS 10 par la société SEPPIC.

Parmi les pigments que l'on peut associer à la composition (C₁) objet de la présente invention, on peut citer le dioxyde de titane, les oxydes de fer marrons, les oxydes de fer jaune, les oxydes de fer noir, ou les oxydes de fer rouges ou encore les pigments nacrés blancs ou colorés tels que les Mica-Titane.

Parmi les filtres solaires que l'on peut associer à la composition (C₁) objet de la présente invention, on peut citer tous ceux figurant dans la directive cosmétique 76/768/CEE modifiée annexe VII, comme par exemple l'oxyde de titane, l'oxyde de zinc, les esters de l'acide cinnamique comme par exemple le 4-méthoxy cinnamate de 2-éthyl hexyle, le 4-méthoxycinnamate d'isopentyle, les dérivés non ioniques de benzophénone, les esters de l'acide 4-amino benzoïque comme par exemple le 4-(diméthylamino) benzoate de 2-éthyl hexyle, le 4-(diméthylamino) benzoate d'amyle.

Selon un autre aspect particulier, l'invention a pour objet une composition (C₁) telle que définie précédemment, caractérisée en ce que sa viscosité dynamique mesurée à une température de 20°C, au moyen d'un viscosimètre de type Brookfield est supérieure ou égale à 30.000 mPa.s et inférieure ou égale à 200.000mPa.s, plus particulièrement supérieure ou égale à 40.000 mPa.s et inférieure ou égale à 130.000 mPa.s, et encore plus particulièrement supérieure ou égale à 50.000 mPa.s et inférieure ou égale à 130.000mPa.s.

Lorsque la viscosité dynamique de la composition (C₁) est inférieure ou égale à environ 100.000mPa.s à une température de 20°C, ladite viscosité dynamique est mesurée au moyen d'un viscosimètre de type Brookfield LVT à une vitesse de 6 tours par minutes.

Lorsque la viscosité dynamique de la composition (C₁) est supérieure à environ 100.000 mPa.s à une température de 20°C, ladite viscosité dynamique est mesurée au moyen d'un viscosimètre de type Brookfield RVT à une vitesse de 5 tours par minute.

Selon un aspect particulier, l'invention a pour objet une composition (C₁) telle que définie précédemment, caractérisée en ce que la conductivité de ladite composition (C₁), mesurée à une température de 20°C au moyen d'un conductimètre de marque LF 196 de la société WTW muni d'une électrode Tétracon 96, est supérieure ou égale à 15 millisiemens.cm⁻¹ (mS.cm⁻¹) et inférieure ou égale à 200 mS.cm⁻¹, plus particulièrement supérieure ou égale à 15 mS.cm⁻¹et inférieure ou égale à 150 mS.cm⁻¹.

La composition (C₁) objet de la présente invention se présente notamment sous la forme d'une émulsion ou d'une microémulsion à phase continue aqueuse.

Lorsque la composition (C₁) objet de la présente invention possède des caractéristiques de fluidité appropriées, elle peut aussi servir pour l'imprégnation de supports constitués de fibres textiles synthétiques ou naturelles, tissées ou non tissées, ou de papiers, pour constituer des articles, comme par exemple des lingettes, destinés au soin, à la protection ou au nettoyage de la peau, du cuir chevelu ou des cheveux, ou comme par exemple des papiers a usage sanitaire ou domestique.

La composition (C₁) objet de la présente invention, peut être mise en oeuvre par application sur la peau, les muqueuses, les cheveux ou le cuir chevelu, qu'il s'agisse d'une application directe dans le cas d'une composition cosmétique, dermocosmétique, dermopharmaceutique ou pharmaceutique, ou d'une application indirecte dans le cas d'un produit de soin, de protection, de nettoyage du corps se présentant sous la forme d'un article en textile, comme par exemple une lingette, ou en papier, comme par exemple un papier à usage sanitaire, destines a être en contact avec la peau, les cheveux ou le cuir chevelu.

La composition (C₁) telle que définie ci-dessus et objet de la présente invention, est stable dans le temps après une période de stockage d'au moins un mois à 20°C et conserve un aspect homogène, ne montrant pas l'apparition de grumeaux ou d'amas, à l'issue de cette même période de stockage dans les mêmes conditions expérimentales, sans qu'il soit nécessaire d'incorporer dans ladite composition (C₁) des tensioactifs émulsionnants.

Selon un aspect particulier, la présente invention a pour objet une composition (C₁) comprenant pour 100% de sa masse une quantité de 0% massique d'agents tensioactifs émulsionnants (EM) choisi parmi les éléments du groupe (G₁) constitué par :
- les acides gras comportant de 14 à 22 atomes de carbone,
- les acides gras éthoxylés comportant de 14 à 22 atomes de carbone,
- les esters d'acide gras comportant de 14 à 22 atomes de carbone et de sorbitol,
- les esters d'acide gras comportant de 14 à 22 atomes de carbone et de polyglycérol,
- les alcools gras comportant de 14 à 22 atomes de carbone éthoxylés,
- les esters d'acide gras comportant de 14 à 22 atomes de carbone et de sucrose,
- les alkylpolyglycosides de formule (II) :

   R₃-O-(S)_{z}-H (III)

   dans laquelle z représente un nombre décimal compris entre 1 et 5, S représente le reste d'un sucre réducteur et R₃ représente un radical alkyle linéaire ou ramifié, saturé ou insaturé, ayant de 14 à 22 atomes de carbone, de préférence de 16 à 22 atomes de carbone, ou un mélange de composés de formule (III).

Dans la définition de la formule (III) telle que définie précédemment, z est un nombre décimal qui représente le degré moyen de polymérisation du reste S. Lorsque z est un nombre entier, (S)_{z} est le reste polymérique de rang z du reste S. Lorsque z est un nombre décimal, la formule (III) représente un mélange de composés :
a1 R₃-O-S-H + a2 R₃-O-(S)₂-H + a3 R₃-O-(S)₃-H + ... + aq R₃-O-(S)_{q}-H avec q représentant un nombre entier compris entre 1 et 10 et dans les proportions molaires a1, a2, a3,... aq telles que :
   q=1
   Σ aq = 1 ; a1 >0
   q=10

Dans la formule (III) telle que définie ci-dessus, z est compris entre 1,05 et 5,0 et plus particulièrement entre 1,05 et 2.

Dans la formule (III) telle que définie ci-dessus, R₃ représente par exemple le radical n-tétradécyle, le radical n-hexadécyle, le radical n-octadécyle, le radical n-eicosyle, le radical n-dodécosyle.

Par sucre réducteur, on désigne, dans la définition de la formule (III), les dérivés saccharidiques qui ne présentent pas dans leurs structures de liaison glycosidique établie entre un carbone anomérique et l'oxygène d'un groupement acétal tels qu'ils sont définis dans l'ouvrage de référence « Biochemistry », Daniel Voet/Judith G. Voet, p. 250, John Wyley & Sons, 1990. La structure oligomérique (S)_{z}, peut se présenter sous toute forme d'isomérie, qu'il s'agisse d'isomérie optique, d'isomérie géométrique ou d'isomérie de position ; elle peut aussi représenter un mélange d'isomères.

Dans la formule (III) telle que définie ci-dessus, le groupe R₃-O- est lié à S par le carbone anomérique du reste saccharide, de manière à former une fonction acétal.

Dans la formule (III) telle que définie ci-dessus, S représente le reste d'un sucre réducteur choisi parmi le glucose, le xylose ou l'arabinose.

Selon un autre aspect particulier, la présente invention a pour objet une composition (C₁) comprenant pour 100% de sa masse de 0,1% à 10% massique, plus particulièrement de 0,1% à 5% massique, et encore plus particulièrement de 0,5% à 3% massique d'au moins un agent tensioactif émulsionnant (EM) choisi parmi les éléments du groupe (G₁) tel que défini ci-dessus.

Selon cet autre aspect particulier, le ratio massique entre la somme de la quantité massique de polyélectrolyte anionique (PA) et la quantité massique du galactomannane (GM), tels que définis précédemment, et la quantité massique de l'agent émulsionnant (EM) est supérieur ou égal à 1,0, plus particulièrement supérieur ou égal à 5,0, et encore plus particulièrement supérieur ou égal à 10,0.

Selon un autre aspect, la présente invention a pour objet un procédé de préparation de la composition (C₁) telle que définie précédemment, caractérisé en ce qu'il comprend :
Au moins une étape a) de préparation d'une phase (P'₁) par mélange du polyélectrolyte anionique réticulé (PA) et du galactomannane (GM) dans la phase grasse (P₁) ; et
Au moins une étape b) d'émulsification de la phase (P'₁) obtenue à l'issue de l'étape a) avec la phase aqueuse (P₂) cosmétiquement acceptable.

Dans le procédé objet de l'invention, la phase grasse (P₁) comprend une ou plusieurs huiles et/ou une ou plusieurs cires telles que définies précédemment.

Dans le cas où la phase grasse (P₁) n'est pas constituée par une seule huile ou par une seule cire, la phase grasse (P₁) est préparée par mélange des ingrédients la constituant à une température typiquement comprise entre 20°C et 85°C, et encore plus particulièrement à une température comprise entre 20°C et 60°C, et par le moyen de tout dispositif de mélange connu de l'homme du métier, comme par exemple par le moyen d'un dispositif d'agitation mécanique équipé d'un mobile de type « ancre », à des vitesses d'agitation comprises entre 50 tours par minute et 500 tours par minutes, plus particulièrement entre 50 tours par minute et 300 tours par minutes.

Dans le procédé objet de l'invention, l'étape a) de préparation d'une phase (P'₁) par mélange du polyélectrolyte anionique réticulé (PA) et du galactomannane (GM) dans la phase grasse (P₁) peut être avantageusement mise en oeuvre à une température inférieure ou égale à 85°C et supérieure ou égale à 20°C, plus particulièrement à une température inférieure ou égale à 60°C et supérieure ou égale à 20°C.

Dans le procédé objet de l'invention, l'étape a) de préparation d'une phase (P'₁) par mélange du polyélectrolyte anionique réticulé (PA) et du galactomannane (GM) dans la phase grasse (P1) peut être réalisée par le moyen de tout dispositif de mélange connu de l'homme du métier, comme par exemple le moyen d'un dispositif d'agitation mécanique équipé d'un mobile de type « ancre », à des vitesses d'agitation comprises entre 50 tours par minute et 500 tours par minutes, plus particulièrement entre 50 tours par minute et 300 tours par minutes, et comme par exemple par le moyen d'un dispositif d'agitation de type rotor-stator à des vitesses d'agitation comprises entre 100 tours par minute et 10.000 tours par minutes, plus particulièrement entre 500 tours par minutes et 4.000 tours par minutes.

Dans le procédé objet de l'invention, l'étape b) d'émulsification de la phase (P'₁) obtenue à l'issue de l'étape a) avec la phase aqueuse (P₂).peut être avantageusement mise en oeuvre à une température comprise entre 20°C et 90°C, plus particulièrement à une température comprise entre 20°C et 85°C, et encore plus particulièrement à une température comprise entre 20°C et 60°C.

Dans le procédé objet de l'invention, l'étape b) d'émulsification de la phase (P'₁) obtenue à l'issue de l'étape a) avec la phase aqueuse (P₂) peut être réalisée par le moyen de tout dispositif de mélange connu de l'homme du métier, comme par exemple le moyen d'un dispositif d'agitation mécanique équipé d'un mobile de type « ancre », à des vitesses d'agitation comprises entre 50 tours par minute et 500 tours par minute, plus particulièrement entre 50 tours par minute et 300 tours par minute, et comme par exemple par le moyen d'un dispositif d'agitation de type rotor-stator a des vitesses d'agitation comprises entre 100 tours par minute et 10.000 tours par minute, plus particulièrement entre 500 tours par minute et 4.000 tours par minute.

Dans le procédé objet de l'invention, la phase aqueuse cosmétiquement acceptable (P₂) comprend de l'eau, et éventuellement un ou plusieurs solvants organiques cosmétiquement acceptables tels que décrits précédemment, et de 1% à 25% massique, pour 100% de la masse de ladite phase aqueuse (P₂) cosmétiquement acceptable d'au moins un sel (S) se présentant sous une forme dissoute et tel que défini précédemment.

La phase aqueuse (P₂) cosmétiquement acceptable est préparée par mélange de l'eau, et éventuellement d'un ou plusieurs solvants organiques cosmétiquement acceptable, avec au moins un sel (S) tel que décrit précédemment, à une température comprise entre 20°C et 85°C, et encore plus particulièrement à une température comprise entre 20°C et 60°C, et par le moyen de tout dispositif de mélange connu de l'homme du métier, comme par exemple par le moyen d'un dispositif d'agitation mécanique équipé d'un mobile de type « ancre », à des vitesses d'agitation comprises entre 50 tours par minute et 500 tours par minutes, plus particulièrement 20 entre 50 tours par minute et 300 tours par minutes.

Selon un autre aspect, la présente invention a pour objet l'utilisation cosmétique de la composition (C₁) telle que définie précédemment, pour le nettoyage, pour la protection et/ou pour le soin de la peau, des cheveux, du cuir chevelu ou des muqueuses.

Dans le cadre de la présente invention, par « utilisation cosmétique » on désigne les utilisations de la composition (C₁) destinées à améliorer et/ou préserver l'aspect esthétique extérieur de la peau, des cheveux, du cuir chevelu ou des muqueuses.

Selon un aspect plus particulier, la composition (C₁) objet de la présente invention peut être utilisée pour le nettoyage de la peau, des muqueuses, des cheveux ou du cuir chevelu, et plus particulièrement peut être utilisée comme gel pour le bain ou pour la douche, comme shampooing. Dans cette utilisation particulière, elle comprend en outre au moins un tensioactif détergent non ionique tel que décrit précédemment.

Selon un autre aspect plus particulier, la composition (C₁) objet de la présente invention, peut être utilisée pour le soin ou pour la protection de la peau, comme par exemple comme crème, comme lait ou comme lotion pour le soin ou pour la protection du visage, des mains et du corps.

Selon cet aspect particulier, la composition (C₁) objet de la présente invention, peut aussi être utilisée plus particulièrement comme produit protecteur de la peau contre les rayons du soleil, comme produit de maquillage de la peau, comme produit protecteur de la peau contre le vieillissement cutané, comme produit hydratant de la peau, comme produit de traitement cosmétique de l'acné et/ou des points noirs et/ou des comédons.

Les exemples suivants illustrent l'invention sans toutefois la limiter.

### 1-1 Préparation d'un terpolymère de 2-méthyl 2-[(1-oxo 2-propènyl) aminol 1-propanesulfonate d'ammonium, de N,N-diméthyl acrylamide et de méthacrylate de lauryle tétraéthoxylé [AMPSNH₄/DMAM/MAL(4OE) 77,4/19,2/3,4 molaire], réticulé au propanetriacrylate de triméthylol (TMPTA) [Exemple selon l'invention].

On charge dans un réacteur maintenu à 25°C sous agitation, 592g d'une solution aqueuse à 15% massique de 2-méthyl 2-[(1-oxo 2-propènyl) amino] 1-propanesulfonate d'ammonium (AMPSNH₄) dans un mélange tert-butanol/eau (97,5/2,5 en volume), 10,1g de N,N-diméthyl acrylamide (DMAM), 4,2g de méthacrylate de lauryle tétraéthoxylé [MAL(4OE)] et 0,75g de TMPTA.

Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C.

Après refroidissement, la poudre qui s'est formée en cours de polymérisation, est filtrée et séchée pour obtenir le produit désiré, dénommé par la suite : "Polyélectrolyte PA₁".

### 2-1 : Préparation d'émulsions huile-dans-eau selon l'invention.

On prépare six émulsions huile-dans-eau selon l'invention, notées (E₁) à (E₆), dont les proportions massiques en leurs constituants sont consignées dans le tableau 1 ci-dessous, en mettant en oeuvre le procédé suivant :
- Dans un premier bécher, on disperse progressivement et successivement à une température de 20°C, le polyélectrolyte PA₁ et la gomme de Guar dans une phase grasse sous agitation mécanique à 80 tours par minute ;
- On prépare dans un second bécher, à une température de 20°C, la phase aqueuse comprenant l'eau et la quantité massique de sel souhaitée ;
- Le contenu du premier bécher est progressivement versé dans le second bécher à une température de 20°C, sous agitation mécanique au moyen d'une défloculeuse à 1.200 tours par minute ;
- Le mélange obtenu est maintenu sous agitation pendant 10 minutes, puis vidangé pour obtenir les émulsions huile-dans-eau (E₁) à (E₆).

**Tableau 1**

| **Emulsion** | **(E₁)** | (**E₂**) | **(E₃)** | **(E₄)** | **(E₅)** | **(E₆)** |
|---|---|---|---|---|---|---|
| **Phase grasse :** | | | | | | |
| Triglycérides C8-C10 | 15% | 15% | 15% | 15% | 15% | 15% |
| **Système stabilisant :** | | | | | | |
| Polyélectrolyte (PA₁) | 1% | 1% | 1% | 1,25% | 0,5% | 1,50% |
| Gomme de Guar⁽²⁾ | 1% | 1% | 1% | 0,75% | 1,50% | 0,50% |
| **Phase aqueuse :** | | | | | | |
| Eau | Qs.100% | Qs.100% | Qs.100% | Qs.100% | Qs.100% | Qs.100% |
| Chlorure de sodium | 2% | 4% | 6% | 8% | 10% | 2% |
| Geogard™ 221⁽¹⁾ | 0,6% | 0,6% | 0,6% | 0,6% | 0,6% | 0,6% |

| | | | | | | |
|---|---|---|---|---|---|---|
| (1) : Geogard™ 221 est un mélange d'acide déhydroacétique et d'alcool benzylique utilisé comme agent conservateur et commercialisé par la société LONZA. (2) : Gomme de Guar (numéro CAS : 9000-30-0) est commercialisée sous l'appellation « VIDOCREM™ E » par la société UNIPEKTIN Ingredients AG. | | | | | | |

### 2-2 : Préparation d'émulsions huile-dans-eau selon l'état de la technique.

On prépare six émulsions huile-dans-eau, notées (F₁) à (F₆), dont les proportions massiques en leurs constituants sont consignées dans le tableau 2 ci-dessous, en mettant en oeuvre le procédé suivant :
- Dans un premier bécher, on disperse progressivement et successivement à une température de 20°C, le polyélectrolyte PA₁ et la gomme de Guar dans une phase grasse sous agitation mécanique à 80 tours par minute ;
- On prépare dans un second bécher, à une température de 20°C, la phase aqueuse comprenant l'eau et la quantité massique de sel souhaitée ;
- Le contenu du premier bécher est progressivement versé dans le second bécher à une température de 20°C, sous agitation mécanique au moyen d'une défloculeuse à 1.200 tours par minute ;
- Le mélange obtenu est maintenu sous agitation pendant 10 minutes, puis vidangé pour obtenir les émulsions huile-dans-eau (F₁) à (F₆).

**Tableau 2**

| **Emulsion** | **(F₁)** | **(F₂)** | **(F₃)** | **(F₄)** | **(F₅)** | **(F₆)** |
|---|---|---|---|---|---|---|
| **Phase grasse :** | | | | | | |
| Triglycérides C8-C10 | 15% | 15% | 15% | 15% | 15% | 15% |
| **Système stabilisant** : | | | | | | |
| Polyélectrolyte (PA₁) | 1% | 2% | 2% | 0% | 1,25% | 0,5% |
| Gomme de Guar | 1% | 0% | 0% | 2% | 0,75% | 1,50% |
| **Phase aqueuse :** | | | | | | |
| Eau | Qs.100% | Qs.100% | Qs.100% | Qs.100% | Qs.100% | Qs.100% |
| Chlorure de sodium | 0% | 2% | 4% | 4% | 0% | 0% |
| Geogard™ 221 | 0,6% | 0,6% | 0,6% | 0,6% | 0,6% | 0,6% |

### 2-3 : Mise en évidence des propriétés et des caractéristiques des émulsions huile-dans-eau selon l'invention comparativement aux émulsions huile-dans-eau de l'état de la technique.

Les émulsions huile-dans-eau (E₁) à (E₆) selon l'invention et les émulsions huile-dans-eau (F₁) à (F₆) selon l'état de la technique ainsi préparées, sont ensuite conservées dans une enceinte climatique isolée et régulée à une température de 20°C pendant 7 jours. A l'issue de cette période de 7 jours et pour chaque émulsions huile-dans-eau :
- On observe l'aspect visuel,
- On mesure la viscosité dynamique (µ) à 20°C, au moyen d'un viscosimètre de type Brookfield LVT à une vitesse de 6 tours par minute (V6) lorsque ladite viscosité dynamique est inférieure ou égale à 100.000 mPa.s environ, muni du mobile adapté ou au moyen d'un viscosimètre de type Brookfield RVT à une vitesse de 5 tours par minute lorsque ladite viscosité dynamique est supérieure à 100.000 mPa.s, muni d'un mobile adapté, de chaque émulsion.
- On mesure la conductivité à 20°C, au moyen d'un conductimètre de marque LF 196 de la société WTW muni d'une électrode Tétracon™ 96.

Les émulsions huile-dans-eau sont ensuite replacées et conservées dans la même enceinte climatique isolée et régulée à une température de 20°C jusqu'à trois mois. Après une période de trois mois, chaque émulsion est sortie de l'enceinte climatique pour en observer l'aspect. Les résultats obtenus pour les émulsions huile-dans-eau (E₁) à (E₆) selon l'invention sont consignés dans le tableau 3 ci-après et les résultats obtenus pour les émulsions huile-dans-eau comparatives (F₁) à (F₆) sont consignés dans le tableau 4 suivant.

**Tableau 3**

| **Emulsion** | **(E₁)** | **(E₂)** | **(Es)** | **(E₄)** | **(E₅)** | **(E₆)** |
|---|---|---|---|---|---|---|
| Aspect visuel après 7 jours à 20°C | ++ | ++ | ++ | ++ | ++ | ++ |
| Viscosité (Brookfield LVT, V6) en mPas. | 59.000 | 51.400 | 57.000 | 43.000 | 73.500 | 41.000 |
| Conductivité en mS.cm⁻¹ | 32,3 | 57,5 | 118,7 | 58,2 | 56,6 | 58,0 |
| Aspect visuel après 3 mois à 20°C | ++ | ++ | ++ | ++ | ++ | ++ |

| | | | | | | |
|---|---|---|---|---|---|---|
| ++ : Aspect homogène et lisse | | | | | | |

**Tableau 4**

| **Emulsion** | **(F₁)** | **(F₂)** | **(F₃)** | **(F₄)** | **(F₅)** | **(F₆)** |
|---|---|---|---|---|---|---|
| Aspect visuel après 7 jours à 20°C | (-) | (-) | (-) | (-) | (-) | (-) |
| Viscosité (Brookfield LVT, V6) en mPas. | 175.000* | 72.500 | 55.000 | 32000 | 83500 | 89000 |
| Conductivité en mS.cm⁻¹ | 1,4 | 31,6 | 57,0 | 55,4 | 1,7 | 0,7 |
| Aspect visuel après 3 mois à 20°C | (--) | (--) | (--) | (--) | (--) | (--) |

| | | | | | | |
|---|---|---|---|---|---|---|
| * : viscosité dynamique mesurée à 20°C avec le viscosimètre Brookfield RVT, vitesse 5 tours par minute. (-) : Présence de grumeaux et d'amas (--) : Aspect hétérogène avec présence de grumeaux et d'amas | | | | | | |

### 2-4 : Analyse des résultats.

Les résultats sont jugés satisfaisants lorsque l'aspect visuel d'une émulsion huile-dans-eau est jugé homogène et lisse après une durée de stockage de trois mois à 20°C de ladite émulsion huile-dans-eau, et lorsque sa viscosité dynamique mesurée à 20°C, au moyen d'un viscosimètre de type Brookfield LVT à une vitesse de 6 tours par minutes, muni du mobile adapté, est supérieure ou égale à 30.000mPa.s.

Les émulsions (E₁) à (E₆) selon l'invention présentent un aspect lisse, dépourvu de grumeaux et d'amas, même à l'issu d'une période de stockage prolongée de 3 mois à 20°C.

Les résultats obtenus avec les émulsions (F₂) et (F₃), font apparaître que, lorsque le système stabilisant de l'émulsion huile-dans-eau est constitué du seul polyélectrolyte (PA₁) en présence d'une quantité de 2% et de 4% de chlorure de sodium, on n'obtient pas d'émulsions huile-dans-eau présentant un aspect homogène et lisse après une période de stockage de 7 jours à 20°C.

De plus les résultats obtenus avec l'émulsion (F₄) font apparaître que lorsque le système stabilisant de l'émulsion huile-dans-eau est constitué de la seule gomme de Guar en présence de 4% de NaCl, on n'obtient pas d'émulsions huile-dans-eau présentant un aspect homogène et lisse après une période de stockage de 7 jours à 20°C.

Pour des ratios massiques «gomme de Guar » sur polyélectrolyte (PA₁) respectivement égaux à 1/1, à 3/5 et à 3/1 et en absence de chlorure de sodium, les résultats obtenus avec les émulsions (F₁), (F₅) et (F₆) font apparaître que l'on n'obtient pas d'émulsions huile-dans-eau présentant un aspect homogène et lisse après une période de stockage de 7 jours à 20°C.

La comparaison des résultats obtenus avec les émulsions huile-dans-eau (E₁) à (E₆) selon l'invention avec ceux obtenus avec les émulsions huile-dans-eau (F₁) à (F₆) selon l'état de la technique, permet de mettre en évidence une amélioration de l'aspect des émulsions huile-dans-eau riches en sel, et tout en conservant un niveau élevé de viscosité, constitutive d' un effet technique supplémentaire induit par l'invention objet de la présence demande de brevet.

### Exemple de formules illustratives

### 3-1 : Gel-crème soin hydratant corporel

**FORMULE :**

| | | |
|---|---|---|
| A | Huile de Jojoba | 14,10% |
| | C12-C15 alkyl Benzoate | 6,7% |
| | DC 245 | 4,2% |
| | DL alpha Tocophérol | 0,05% |
| B | Maris Aqua | 70,85% |
| | AQUAXYL™ | 3% |
| C | Polyélectrolyte (PA₁) | 2% |
| | Gomme de Guar⁽²⁾ | 1% |
| D | Euxyl™ PE9010 | 1% |
| | Fragrance | 0,1% |

### MODE OPERATOIRE :

Mélanger les constituants de la phase grasse A à une température de 80°C sous agitation. Y ajouter ensuite successivement les ingrédients de la phase C.

Préparer la phase aqueuse B et la chauffer à 80°C sous agitation.

Ajouter la phase aqueuse B de façon progressive sur le mélange des phases A+C puis émulsionner à l'aide d'un agitateur muni d'un mobile rotor-stator de marque Silverson. Refroidir ensuite à 25°C, puis ajouter la phase D.

### 3-2: Gel-crème Masque visage.

**FORMULE :**

| | | |
|---|---|---|
| A | Triglycérides 4555 (C8C10) | 9% |
| | C12-C15 alkyl Benzoate | 4% |
| | Isohexadécane | 2% |
| | DL alpha Tocopherol | 0,10% |
| B | Maris Aqua | qsp 100% |
| C | Polyélectrolyte (PA₁) | 1,3% |
| | Gomme de Guar⁽²⁾ | 0,7% |
| D | Euxyl PE9010 | 1% |
| | Fragrance | 0,1% |

### MODE OPERATOIRE :

Mélanger les constituants de la phase grasse A à une température de 80°C sous agitation. Y Ajouter ensuite successivement les ingrédients de la phase C.

Préparer la phase aqueuse B et la chauffer à 80°C sous agitation.

Ajouter la phase aqueuse B de façon progressive sur le mélange des phases A+C puis émulsionner à l'aide d'un agitateur muni d'un mobile rotor-stator de marque Silverson. R

Refroidir ensuite à 25°C et ajouter la phase D.

### 3-3 : Crème corporelle.

**FORMULE :**

| | |
|---|---|
| Triglycérides 4555 (C8C10) | 12% |
| C12-C15 alkyl Benzoate | 5,3% |
| Isohexadécane | 2,7% |
| Alcool cétylique | 2% |
| DL alpha Tocophérol | 0,10% |
| Polyélectrolyte (PA₁) | 1,5% |
| Gomme de Guar⁽²⁾ | 0,5% |
| Eau | qsp 100% |
| Givobio™ GZn | 1% |
| Sepicalm™ S | 3% |
| Euxyl™ PE9010 | 1% |
| Fragrance | 0,1% |

### 3-4 : Spray solaire organo-minéral

**FORMULE:**

| | | |
|---|---|---|
| A | Isodecyl neopentanoate | 20% |
| | Cyclodiméthicone | 5% |
| | Ethylhexylmethoxicinnamate | 6% |
| | Butyl Methoxydibenzoylmethane | 3% |
| | DL alpha Tocopherol | 0.05% |
| B | Eau | qsp 100% |
| | EDTA tétrasodique | 0,2% |
| | Glycérine | 7% |
| | Phenyl Benzyimidazole Sulfonic Acid | |
| (salifié avec quantité molaire nécessaire de soude) | | 3% |
| C | Polyélectrolyte (PA₁) | 1,3% |
| | Gomme de Guar⁽²⁾ | 0,7% |
| D | SEPICIDE™ HB | 1% |
| | Fragrance | 0,1% |

AQUAXYL™ (nom INCI : Xylitylglucoside & Anhydroxylitol & Xylitol) : Composition hydratante commercialisée par la société SEPPIC.
Euxyl™ PE9010 (Nom INCI : Phenoxyethanol & Ethylhexyl Glycerin) : Composition utilisée comme agent conservateur.
GIVOBIO™ GZn (nom INCI : Zinc Gluconate) : Composition commercialisée par la société SEPPIC.
LANOL™ 99 (nom INCI : Isononyle Isononanoate) : Ester utilisé comme phase huileuse dans la préparation de composition cosmétique et distribué par la société SEPPIC.
Maris Aqua : eau de mer à 8% de chlorure de sodium.
SEPICIDE™ HB (nom INCI: Phenoxyethanol / Methylparaben / Ethylparaben / Propylparaben / Butylparaben) : Agent conservateur contenant du phénoxyéthanol, commercialisé par la société SEPPIC.
SEPICALM™ S : (nom INCI : Sodium Cocoyl Aminoacids And Sarcosine And Potassium Aspartate And Magnésium Aspartate) : Composition anti-inflammatoire commercialisée par la société SEPPIC.
SERENIKS™ 207 (nom INCI : Tsuga Canadensis Leaf Extract And Water And Butylene Glycol) est une composition anti-âge.

## Revendications

1. Composition (C₁) se présentant sous la forme d'une émulsion de type huile-dans-eau, **caractérisée en ce qu'**elle comprend pour 100% de sa masse :
- de 5% à 55% massique d'une phase grasse (P₁) constituée d'au moins une huile et/ou d'au moins une cire,
- de 0,025% à 3,75% massique d'au moins un polyélectrolyte anionique réticulé (PA) issu de la polymérisation de l'acide 2-méthyl 2-[(1-oxo 2-propènyl) amino] 1-propanesulfonique , partiellement ou totalement salifié, avec au moins un monomère neutre choisi parmi l'acrylamide, l'acrylate de (2-hydroxy éthyle) ou le N,N-diméthyl acrylamide, et au moins un monomère de formule (I) : 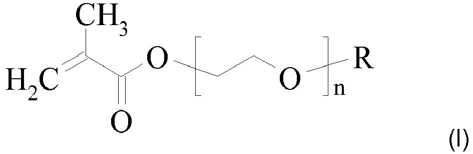 dans laquelle R représente un radical alkyle linéaire ou ramifié comportant de huit à vingt atomes de carbone et n représente un nombre supérieur ou égal à un et inférieur ou égal à vingt, en présence d'au moins un agent de réticulation,
- de 0,025% à 3,75% massique d'au moins un galactomannane (GM) présentant un degré de substitution (DS) d'environ 1/2,
- de 37,5% à 94,95% massique d'une phase aqueuse (P₂) cosmétiquement acceptable, ladite phase aqueuse (P₂) comprenant pour 100% de sa masse de 1% à 25% massique d'au moins un sel (S) se présentant sous une forme dissoute,
ladite composition (C₁) étant en outre **caractérisée en ce que** le rapport massique entre le galactomannane (GM) et le polyélectrolyte anionique réticulé (PA) est supérieur ou égal à 1/3 et inférieur ou égal à 3/1.

2. Composition (C₁) telle que définie à la revendication 1, **caractérisée en ce que** ledit polyélectrolyte anionique réticulé (PA) comporte pour 100% molaire de ses monomères constitutifs :
- de 20% molaire à 80% molaire d'unités monomériques issues d'un monomère comportant une fonction acide fort partiellement ou totalement salifiée ;
- de 15% molaire à 75% molaire d'unités monomériques issues d'un monomère neutre ;
- de 0,5% à 5% molaire d'unités monomériques issues d'un monomère de formule (I) telle que définie précédemment.

3. Composition (C₁) telle que définie à l'une ou quelconque des revendications 1 ou 2, **caractérisée en ce que** dans ledit polyélectrolyte anionique réticulé (PA) ledit monomère de formule (I) est le méthacrylate de lauryle tétraéthoxylé.

4. Composition (C₁) telle que définie à l'une ou quelconque des revendications 1 à 3, **caractérisée en ce que** ledit polyélectrolyte anionique réticulé (PA) est un terpolymère de l'acide 2-méthyl 2- [(1-oxo 2-propènyl) amino] 1-propanesulfonique partiellement salifié sous forme de sel d'ammonium, du N,N-diméthyl acrylamide et du méthacrylate de lauryle tétraéthoxylé, réticulé au triméthylol propanetriacrylate.

5. Composition (C₁) telle que définie à l'une quelconque des revendications 1 à 4, **caractérisée en ce que** le sel (S) est un sel inorganique constitué par un cation qui est l'ion ammonium ou un cation métallique et par un anion sélectionné parmi les éléments du groupe constitué par les halogénures, les carbonates, les bicarbonates , les phosphates, les nitrates, les borates et les sulfates.

6. Composition (C₁) telle que définie à l'une quelconque des revendications 1 à 5, **caractérisée en ce que** le sel (S) est un sel organique constitué par un cation qui est l'ion ammonium ou un cation métallique et par un anion organique qui est un composé organique possédant au moins une fonction acide carboxylique sous forme carboxylate ou au moins une fonction acide sulfonique sous forme sulfonate ou au moins une fonction sulphate .

7. Composition (C₁) telle que définie à la revendication 6, **caractérisée en ce que** le sel (S) est un sel organique sélectionné parmi les éléments du groupe constitué par le glycolate de sodium, le citrate de sodium, le salicylate de sodium, le lactate de sodium, le gluconate de sodium, le gluconate de zinc, le gluconate de manganèse, le gluconate de cuivre et l'aspartate de magnésium.

8. Composition (C₁) telle que définie à la revendication 6, **caractérisée en ce que** le sel (S) est un sel organique sélectionné parmi les éléments du groupe constitué par le 2-phényl benzimidazole-5-sulphonate de sodium et le 4-hydroxy 2-méthoxy-5-(oxo-phénylméthyl) benzènesulphonate de sodium.

9. Composition (C₁) telle que définie à l'une ou quelconque des revendications 1 à 8, **caractérisée en ce que** sa viscosité dynamique mesurée à une température de 20°C, au moyen d'un viscosimètre de type Brookfield est supérieure ou égale à 30.000 mPa.s et inférieure ou égale à 200.000 mPa.s

10. Procédé de préparation d'une composition (C₁) telle que définie à l'une ou quelconque des revendications 1 à 9, **caractérisé en ce qu'**il comprend :
Au moins une étape a) de préparation d'une phase (P'₁) par mélange du polyélectrolyte anionique réticulé (PA) et du galactomannane (GM) dans la phase grasse (P₁) ; et
Au moins une étape b) d'émulsification de la phase (P'₁) obtenue à l'issue de l'étape a) avec la phase aqueuse (P₂) cosmétiquement acceptable.

11. Utilisation cosmétique de la composition (C₁) telle que définie à l'une ou quelconque des revendications 1 à 9 pour le nettoyage, pour la protection et/ou pour le soin de la peau, des cheveux, du cuir chevelu ou des muqueuses.

## Patentansprüche

1. Zusammensetzung (C₁), die die Form einer Emulsion vom Typ Öl-in-Wasser aufweist, **dadurch gekennzeichnet, dass** sie bezogen auf 100 % ihrer Masse Folgendes umfasst:
- von 5 bis 55 Massenprozent einer Fettphase (P₁), die aus mindestens einem Öl und/oder mindestens einem Wachs besteht,
- von 0,025 bis 3,75 Massenprozent mindestens eines vernetzten anionischen Polyelektrolyts (PA), das aus der Polymerisierung der 2-Methyl 2-[(1-oxo 2-propenyl) amino]1-propansulfonsäure stammt, teilweise oder vollständig salzhaltig, mit mindestens einem neutralen Monomer, ausgewählt aus Acrylamid, (2-Hydroxyethyl)acrylat oder N,N-Dimethylacrylamid und mindestens einem Monomer mit der folgenden Formel (I): 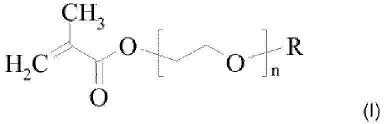 wobei R ein lineares oder verzweigtes Alkylradikal darstellt, umfassend von acht bis zwanzig Kohlenstoffatome, und n eine Anzahl darstellt, die größer als oder gleich eins und kleiner als oder gleich zwanzig ist, in Anwesenheit von mindestens einem Vernetzungsmittel,
- von 0,025 bis 3,75 Massenprozent mindestens eines Galactomannans (GM), das einen Substitutionsgrad (DS) von ungefähr 1/2 aufweist,
- von 37,5 bis 94,95 Massenprozent einer kosmetisch annehmbaren wässrigen Phase (P₂), wobei die wässrige Phase (P₂) bezogen auf 100 % ihrer Masse von 1 bis 25 Massenprozent mindestens eines Salzes (S) umfasst, das eine gelöste Form aufweist,
wobei die Zusammensetzung (C₁) außerdem **dadurch gekennzeichnet ist, dass** das Massenverhältnis zwischen dem Galactomannnan (GM) und dem vernetzten anionischen Polyelektrolyten (PA) größer als oder gleich wie 1/3 und kleiner als oder gleich wie 3/1 ist.

2. Zusammensetzung (C₁) nach Anspruch 1, **dadurch gekennzeichnet, dass** der vernetzte anionische Polyelektrolyt (PA) bezogen auf 100 Molprozent seiner konstitutiven Monomere Folgendes umfasst:
- von 20 Molprozent bis 80 Molprozent Monomereinheiten, die von einem Monomer stammen, das eine starke, teilweise oder vollständig salzhaltige Säurefunktion umfasst;
- von 15 bis 75 Molprozent Monomereinheiten, die von einem neutralen Monomer stammen;
- von 0,5 bis 5 Molprozent Monomereinheiten, die von einem Monomer mit der Formel (I) stammen, wie oben definiert.

3. Zusammensetzung (C₁) nach einem oder einem beliebigen der Ansprüche 1 oder 2, **dadurch gekennzeichnet, dass** im vernetzten anionischen Polyelektrolyt (PA) das Monomer mit der Formel (I) Tetraethoxyllaurylmethacrylat ist.

4. Zusammensetzung (C₁) nach einem oder einem beliebigen der Ansprüche 1 bis 3, **dadurch gekennzeichnet, dass** der vernetzte anionische Polyelektrolyt (PA) ein Terpolymer der 2-Methyl 2-[(1-oxo 2-propenyl)amino]1-propansulfonsäure, teilweise salzhaltig in Form eines Ammoniumsalzes, N,N-Dimethylacrylamid und Tetraethoxyllaurylmethacrylat ist, vernetzt mit Trimethylolpropantriacrylat.

5. Zusammensetzung (C₁) nach einem beliebigen der Ansprüche 1 bis 4, **dadurch gekennzeichnet, dass** das Salz (S) ein anorganisches Salz ist, bestehend aus einem Kation, wobei es sich um das Ammoniumion oder ein metallisches Kation handelt, und aus einem Anion, ausgewählt aus den Elementen der Gruppe, bestehend aus Halogeniden, Carbonaten, Bicarbonaten, Phosphaten, Nitraten, Boraten und Sulfaten.

6. Zusammensetzung (C₁) nach einem beliebigen der Ansprüche 1 bis 5, **dadurch gekennzeichnet, dass** das Salz (S) ein organisches Salz ist, bestehend aus einem Kation, wobei es sich um das Ammoniumion oder ein metallisches Kation handelt, und aus einem organischen Anion, wobei es sich um eine organische Verbindung handelt, die mindestens eine Carbonsäurefunktion in Caboxylatform aufweist oder mindestens eine Sulfonsäurefunktion in Sulfonatform oder mindestens eine Sulfatfunktion.

7. Zusammensetzung (C₁), nach Anspruch 6, **dadurch gekennzeichnet, dass** das Salz (S) ein organisches Salz ist, ausgewählt aus den Elementen der Gruppe, bestehend aus Natriumglycolat, Natriumcitrat, Natriumsalicylat, Natriumlactat, Natriumgluconat, Zinkgluconat, Magnesiumgluconat, Kupfergluconat und Magnesiumaspartat.

8. Zusammensetzung (C₁) nach Anspruch 6, **dadurch gekennzeichnet, dass** das Salz (S) ein organisches Salz ist, ausgewählt aus den Elementen der Gruppe, bestehend aus 2-Phenylbenzimidazol-5-natriumsulphonat und 4-Hydroxy 2-methoxy-5-(oxo-phenylmethyl)natriumbenzensulfonat.

9. Zusammensetzung (C₁) nach einem oder einem beliebigen der Ansprüche 1 bis 8, **dadurch gekennzeichnet, dass** ihre dynamische Viskosität, gemessen bei einer Temperatur von 20 °C mittels eines Viskosimeters vom Typ Brookfield größer als oder gleich wie 30 000 mPa.s und kleiner als oder gleich wie 200 000 mPa.s ist.

10. Verfahren zur Herstellung einer Zusammensetzung (C₁) nach einem oder einem beliebigen der Ansprüche 1 bis 9, **dadurch gekennzeichnet, dass** es Folgendes umfasst:
Mindestens einen Schritt a) des Herstellens einer Phase (P'₁) durch Mischung des vernetzten anionischen Polyelektrolyts (PA) und des Galactomannans (GM) in der Fettphase (P₁); und
Mindestens einen Schritt b) des Emulgierens der Phase (P'₁), die am Ausgang von Schritt a) erhalten wurde, mit der kosmetisch annehmbaren wässrigen Phase (P₂).

11. Kosmetische Verwendung der Zusammensetzung (C₁) nach einem oder einem beliebigen der Ansprüche 1 bis 9 zur Reinigung, zum Schutz und/oder zur Pflege der Haut, der Haare, der Kopfhaut oder der Schleimhäute

## Claims

1. Composition (C₁) in the form of an oil-in-water type emulsion, **characterised in that** it comprises the following for 100% of its mass:
- from 5% to 55% by mass of a fatty phase (P₁) composed of at least one oil and/or at least one wax,
- from 0.025% to 3.75% by mass of at least one cross-linked anionic polyelectrolyte (PA) derived from the polymerisation of 2-methyl 2[(1-oxo 2-propenyl) amino] 1-propanesulfonic acid, partially or totally salified, with at least one neutral monomer chosen from among acrylamide, (2-hydroxy ethyl) acrylate or N,N-dimethyl acrylamide, and at least on monomer with formula (I): 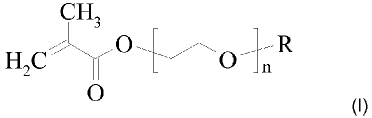 in which R represents a linear or ramified alkyl radical comprising eight to twenty carbon atoms and n represents a number greater than or equal to one and less than or equal to twenty, in the presence of at least one cross-linking agent,
- from 0.025% to 3.75% by mass of at least one galactomannan (GM) with a degree of substitution (DS) equal to about 1/2,
- from 37.5% to 94.95% by mass of a cosmetically acceptable aqueous phase (P₂), said aqueous phase (P₂) comprising for 100% of its mass from 1% to 25% by mass of at least one salt (S) in a dissolved form,
said composition (C₁) also being **characterised in that** the mass ratio between the galactomannan (GM) and the cross-linked anionic polyelectrolyte (PA) is greater than or equal to 1/3 and less than or equal to 3/1.

2. Composition (C₁) as defined in claim 1, **characterised in that** said cross-linked anionic polyelectrolyte (PA) comprises the following for 100% molar of its component monomers:
- from 20% to 80% molar of monomeric units derived from a monomer comprising a partially or totally salified strong acid function,
- from 15% to 75% molar of monomeric units derived from a neutral monomer,
- from 0.5% to 5% molar of monomeric units derived from a monomer with formula (I) as defined above,

3. Composition (C₁) as defined in either claim 1 or 2, **characterised in that** in said cross-linked anionic polyelectrolyte (PA), said monomer with formula (I) is tetraethoxylated lauryl methacrylate.

4. Composition (C₁) as defined in any one of claims 1 to 3, **characterised in that** in said cross-linked anionic polyelectrolyte (PA) is a terpolymer of partially salified 2-methyl 2-[(1-oxo 2-propenyl amino] 1-propanesulfonic acid in the form of ammonium salt, N,N-dimethyl acrylamide and tetraethoxylated lauryl methacrylate, cross-linked with trimethylol propanetriacrylate.

5. Composition (C₁) as defined in any one of claims 1 to 4, **characterised in that** in the salt (S) is an inorganic salt composed of a cation that is the ammonium ion or a metallic cation and by an anion selected from among elements in the group composed of halides, carbonates, bicarbonates, phosphates, nitrates, borates and sulphates.

6. Composition (C₁) as defined in any one of claims 1 to 5, **characterised in that** in the salt (S) is an organic salt composed of a cation that is the ammonium ion or a metallic cation and an organic anion that is an organic compound with at least one carboxylic acid function in carboxylate form or at least one sulfonic acid function in sulphonate form or at least one sulphate function.

7. Composition (C₁) as defined in claim 6, **characterised in that** in the salt (S) is an organic salt selected from among elements of the group composed of sodium glycolate, sodium citrate, sodium salicylate, sodium lactate, sodium gluconate, zinc gluconate, manganese gluconate, copper gluconate and magnesium aspartate.

8. Composition (C₁) as defined in claim 6, **characterised in that** in the salt (S) is an organic salt selected from among elements of the group composed of sodium 2-phenyl benzimidazole-5-sulphonate and sodium 4-hydroxy 2-methoxy-5-(oxo-phenylmethyl) benzenesulphonate.

9. Composition (C₁) as defined in any one of claims 1 to 8, **characterised in that** in its dynamic viscosity measured at a temperature of 20°C using a Brookfield type viscosity meter is greater than or equal to 30 000 mPa.s and less than or equal to 200 000 mPa.s.

10. Method of preparing a composition (C₁) as defined in any one of claims 1 to 9, **characterised in that** it comprises:
At least one step a) to prepare a phase (P'₁) by mixing the cross-linked anionic polyelectrolyte (PA) and galactomannan (GM) in the fatty phase (P₁); and
At least one step b) to emulsify the phase (P'₁) obtained after step a) with the cosmetically acceptable aqueous phase (P₂).

11. Cosmetic use of the composition (C₁) as defined in any one of claims 1 to 9 for cleaning, for protection and/or for care of the skin, hair, scalp and mucous membranes.
